# EUROPÄISCHE PATENTSCHRIFT

(11) **EP 1 908 398 B1**
(45) Veröffentlichungstag und Bekanntmachung des Hinweises auf die Patenterteilung: **05.08.2015**
(21) Anmeldenummer: 07018337.1
(22) Anmeldetag: 19.09.2007
(51) Int. Cl.: A61B 3/13, G02B 21/00

(54) **Ophthalmo-Operationsmikroskopsystem**
Ophthalmic operation microscope system
Système de microscope pour opération ophtalmique

(30) Priorität: 07.10.2006 DE 102006047459
(43) Veröffentlichungstag der Anmeldung: 09.04.2008
(73) Patentinhaber: Carl Zeiss Meditec AG, 07745 Jena (DE)
(72) Erfinder: Gaida, Gerhard, 73430 Aalen (DE)
(74) Vertreter: Gauss, Nikolai

(56) Entgegenhaltungen:
- US-A1- 2002 118 448
- US-A1- 2002 191 280
- US-A1- 2004 184 142
- US-A1- 2005 225 848

## Beschreibung

Die Erfindung betrifft ein Ophthalmo-Operationsmikroskopsystem mit einem Operationsmikroskop, einer Trägereinrichtung, in der das Operationsmikroskop höhenverstellbar aufgenommen ist, um einen Arbeitsabstand zwischen dem Operationsmikroskop und einem Patientenauge einstellen zu können, einem ersten Antrieb zur Höhenverstellung des Operationsmikroskops, einem Ophthalmoskopie-Vorsatzmodul, das eine verstellbare Ophthalmoskopierlupe umfasst, um einen Arbeitsabstand zwischen Ophthalmoskopierlupe und Patientenauge einstellen zu können, sowie einem zweiten Antrieb zur Verstellung der Ophthalmoskopierlupe.

Ein Ophthalmo-Operationsmikroskopsystem der eingangs genannten Art ist aus der DE 202 15 635 U1 bekannt. Dort ist ein Operationsmikroskop beschrieben, das höhenverstellbar in einer Schlitteneinrichtung an einer Stativeinheit aufgenommen ist. Zur Fokussierung kann das Operationsmikroskop mittels einer Antriebseinheit in Form eines Verstellknopfes in der Schlitteneinheit auf und abbewegt werden. Vor dem Mikroskop-Hauptobjektivsystem des Operationsmikroskops ist ein Ophthalmoskopie-Vorsatzmodul angeordnet, das in die Beobachtungsstrahlengänge ein- und ausgeschwenkt werden kann. Das Ophthalmoskopie-Vorsatzmodul ist an das Operationsmikroskop montiert. Das Ophthalmoskopie-Vorsatzmodul trägt eine höhenverstellbare Ophthalmoskopierlupe. Für das Justieren der Ophthalmoskopierlupe ist ein Antrieb vorgesehen, der eine Gewindespindel mit Antriebsmotor umfasst. Um das Ophthalmo-Operationsmikroskopsystem für die Untersuchung des Hintergrunds eines Patientenauges einzustellen, wird das Ophthalmoskopie-Vorsatzmodul in die Beobachtungsstrahlengänge des Operationsmikroskops eingeschwenkt. Dann werden Operationsmikroskop und Ophthalmoskopierlupe so lange bewegt, bis im Binokulartubus des Operationsmikroskops eine scharfe Abbildung des Augenhintergrunds zu sehen ist.

Die Fa. Topcon vertreibt unter der Bezeichnung OMS 800 OFFISS ein Ophthalmo-Operationsmikroskopsystem, das ein Operationsmikroskop mit Ophthalmoskopie-Vorsatzmodul umfasst. Das Operationsmikroskop ist höhenverstellbar an einer Stativeinheit aufgenommen. Getrennt vom Operationsmikroskop ist auch das Ophthalmoskopie-Vorsatzmodul an diese Stativeinheit montiert. Das Ophthalmoskopie-Vorsatzmodul enthält eine Ophthalmoskopierlupe, die in die Beobachtungsstrahlengänge vor dem Hauptobjektivsystem des Operationsmikroskops ein- und ausgeschwenkt werden kann. Die getrennte Aufnahme von Operationsmikroskop und Ophthalmoskopie-Vorsatzmodul an der Stativeinheit ermöglicht, das Operationsmikroskop zu fokussieren, indem es auf- oder abbewegt wird, ohne dass sich gleichzeitig die Position der Ophthalmoskopierlupe verändert.

In US 2004/0184142 A1, US 2002/0191280 A1, US 2005/0225848 A1 und US 2002/011848 A1 ist jeweils ein Operationsmikroskop beschrieben, welches eine Trägereinrichtung, zwei Antriebe und ein Vorsatzmodul mit einer Ophthalmoskopierlupe aufweist.

Aufgabe der Erfindung ist es, ein Ophthalmo-Operationsmikroskopsystem bereitzustellen, das es einem Ophthalmologen ermöglicht, mit einer einzigen Bedieneinheit ein Operationsmikroskop mit Ophthalmoskopie-Vorsatzmodul so einzustellen; dass auch während des Verstellens von Operationsmikroskop und Ophthalmologie-Vorsatzmodul der Augenhintergrund eines Patientenauges scharf, mit gutem Kontrast sowie mit hoher Lichtstärke beobachtet werden kann.

Diese Aufgabe wird durch ein Ophthalmo-Operationsmikroskopsystem gemäß Patentanspruch 1 gelöst, bei dem eine Antriebskopplung vorgesehen ist, die den ersten Antrieb zur Höhenverstellung des Operationsmikroskops mit dem zweiten Antrieb zur Verstellung der Ophthalmoskopierlupe koppelt. Auf diese Weise kann ein Ophthalmologe das Ophthalmo-Operationsmikroskopsystem schnell und bequem für eine Untersuchung des Augenhintergrunds einstellen und fokussieren.

Gemäß der Erfindung ist die Antriebskopplung für den ersten Antrieb zur Höhenverstellung des Operationsmikroskops mit dem zweiten Antrieb zur Verstellung des Ophthalmoskopierlupensystems derart gekoppelt, dass bei Antriebsaktivierung Operationsmikroskop und Ophthalmoskopierlupensystem in entgegengesetzter Richtung bewegt werden. Auf diese Weise wird beim Verstellen des Systems ein gleichbleibender Abstand der Ophthalmoskopierlupe von einem untersuchten Patientenauge gewährleistet. Es ist möglich, die Antriebskopplung vom ersten Antrieb zur Höhenverstellung des Operationsmikroskops und vom zweiten Antrieb zur Verstellung des Ophthalmoskopierlupensystems derart auszubilden, dass Operationsmikroskop und Ophthalmoskopierlupe mit annähernd gleicher Geschwindigkeit bewegt werden.

Gemäß der Erfindung koppelt die Antriebskopplung den ersten Antrieb zur Höhenverstellung des Operationsmikroskops mit dem zweiten Antrieb zur Verstellung der Ophthalmoskopierlupe derart, dass die Gerätepupille des Systems aus Operationsmikroskop und Ophthalmoskopierlupe raumfest ist. Indem die Gerätepupille in die Pupille des Patientenauges gelegt wird, ergibt sich im Operationsmikroskop ein größtmögliches Bildfeld des Augenhintergrunds. Auf diese Weise kann das Ophthalmo-Operationsmikroskopsystem auf den Augenhintergrund festgesetzt werden, ohne dass Vignettierung des Bildfeldes im System auftritt.

In Weiterbildung der Erfindung ist der erste Antrieb als motorischer Antrieb ausgebildet. Auf diese Weise wird einem Ophthalmologen eine bequeme Einstellung des Arbeitsabstands des Operationsmikroskops ermöglicht.

In Weiterbildung der Erfindung ist der zweite Antrieb als motorischer Antrieb ausgebildet. Auf diese Weise wird insbesondere ein fußgesteuertes Bewegen der Ophthalmoskopierlupe ermöglicht.

In Weiterbildung der Erfindung ist die Kopplung als elektronische Kopplung ausgebildet. Dies ermöglicht es, ohne nennenswerten Fertigungsaufwand die Bewegung der Ophthalmoskopierlupe in beliebiger Abhängigkeit von der Position des Operationsmikroskops zu steuern. Insbesondere können so auf einfache Weise nichtlineare Steuerfunktionen realisiert werden.

In Weiterbildung umfasst das Ophthalmoskopie-Vorsatzmodul ein Reduzierlinsensystem. Auf diese Weise wird gewährleistet, dass bei Ausschwenken des Ophthalmoskopie-Vorsatzmoduls aus dem Beobachtungsstrahlengang das Ophthalmo-Operationsmikroskopsystem automatisch auf die Cornea des untersuchten Patientenauges scharfgestellt ist.

In Weiterbildung der Erfindung ist bei dem Ophthalmo-Operationsmikroskopsystem ein schaltbares System zur Strahlvertauschung und Bildumkehr vorgesehen. Auf diese Weise wird gewährleistet, dass der Hintergrund eines Patientenauges im Operationsmikroskop aufrecht sichtbar gemacht werden kann.

In Weiterbildung der Erfindung kann das Ophthalmoskopie-Vorsatzmodul in die Beobachtungsstrahlengänge des Operationsmikroskops ein- und ausgeschwenkt werden. Auf diese Weise wird einer Beobachtungsperson in schneller Folge die abwechselnde Beobachtung von vorderem Abschnitt und Hintergrund eines Patientenauges ermöglicht.

Vorteilhafte Weiterbildung der Erfindung sind in den Figuren dargestellt und werden nachfolgend beschrieben.

Es zeigen:
- Fig. 1: ein erstes Ophthalmo-Operationsmikroskopsystem mit einem in die Beobachtungsstrahlengänge geschwenkten Ophthalmoskopie-Vorsatzmodul;
- Fig. 2: das erste Ophthalmo-Operationsmikroskopsystem, wobei das Ophthalmoskopie-Vorsatzmodul aus den Beobachtungsstrahlengängen geschwenkt ist;
- Fig. 3: ein zweites Ophthalmo-Operationsmikroskopsystem;
- Fig. 4: einen Abschnitt des ersten und zweiten Ophthalmo-Operationsmikroskopsystems;
- Fig. 5: den Abschnitt des Ophthalmo-Operationsmikroskopsystems, wobei die Gerätepupille an die Pupille eines untersuchten Patientenauges angepasst ist; und
- Fig. 6: ein Ophthalmo-Operationsmikroskopsystem, dessen Gerätepupille nicht an die Pupille eines zu untersuchenden Patientenauge angepasst ist.

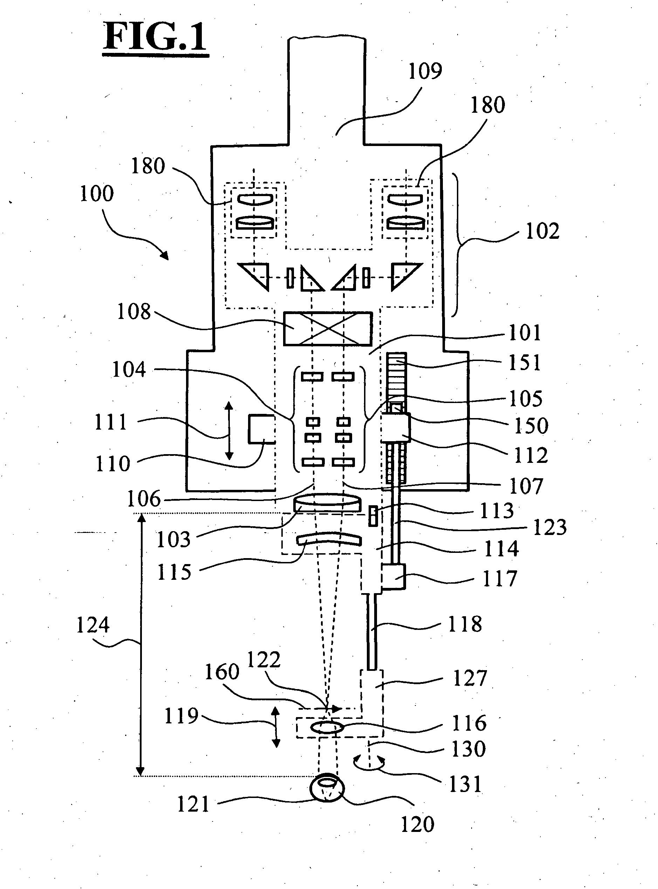

Das Ophthalmo-Operationsmikroskopsystem 100 in Fig. 1 umfasst ein Operationsmikroskop 101, das als Stereomikroskop ausgebildet ist. Das Operationsmikroskop 101 hat einen Einblicktubus 102 mit Okular 180 und ein Hauptobjektivsystem 103. Es enthält ein pankratisches Vergrößerungssystem 104, 105 für einen linken und rechten binokularen Beobachtungsstrahlengang 106, 107. Zwischen dem pankratischen Vergrößerungssystem 104 und dem Einblicktubus 102 ist ein schaltbares System zur Strahlvertauschung und Bildumkehr angeordnet.

Das Operationsmikroskop 101 ist an einer Trägereinrichtung 109 aufgenommen. Es kann daran mit einer Fokussiereinrichtung 110 entsprechend dem Doppelpfeil 111 auf- und ab bewegt werden. Auf diese Weise kann der Arbeitsabstand 124 zwischen dem Mikroskop-Hauptobjektivsystem 103 und einem untersuchten Patientenauge 120 eingestellt werden. Das Operationsmikroskop wird so auf einen Objektbereich, der untersucht werden soll, fokussiert.

Der Fokussiereinrichtung 110 ist ein motorischer Antrieb 112 zugeordnet. Der motorische Antrieb 112 ist mit einem Zahnradgetriebe 150 verbunden, das in eine Zahnstange 151 an der Trägereinrichtung 109 eingreift.

Das Ophthalmo-Operationsmikroskopsystem 100 umfasst weiter ein Ophthalmoskopie-Vorsatzmodul 114. Das Ophthalmoskopie-Vorsatzmodul ist mit einem Schwenkgelenk 113 an das Operationsmikroskop 101 angeschlossen.

Das Ophthalmoskopie-Vorsatzmodul 114 enthält eine Reduzierlinse 115 sowie eine Ophthalmoskopierlupe 116. Die Ophthalmoskopierlupe 116 ist in einer Ophthalmoskopierlupenhalterung 127 aufgenommen. Die Ophthalmoskopierlupenhalterung 127 kann mittels eines Antriebs 117, der auf ein Schneckengewinde 118 wirkt, entsprechend dem Doppelpfeil 119 über einem zu untersuchenden Patientenauge 120 auf- und abbewegt werden. Der Antrieb 112 zur Verstellung des Operationsmikroskops 101 und der Antrieb 117, der die Ophthalmoskopierlupe 116 bewegt, sind über eine Kopplung 123 in Wirkverbindung. Diese Kopplung 123 ist bei dem Ophthalmo-Operationsmikroskopsystem 100 als mechanische Kopplung ausgeführt.

Aufgrund der Brechkraft der Linse 140 in einem Patientenauge ist es für die Untersuchung des Augenhintergrunds 121 eines Patientenauges 120 mit einem Operationsmikroskop 101 erforderlich, den Augenhintergrund 121 in eine Zwischenbildebene 160 abzubilden, auf welche die Beobachtungsstrahlengänge 106, 107 des Operationsmikroskops fokussiert sind. Dies ist die Funktion der Ophthalmoskopierlupe 116. Sie erzeugt in der Zwischenbildebene 160 ein seitenverkehrtes Zwischenbild 122 des Hintergrunds 121 des Patientenauges 120. Um dieses Zwischenbild 122 scharf betrachten zu können, muss die Fokusebene der Beobachtungsstrahlengänge 106, 107 des Operationsmikroskops 101 mit der Zwischenbildebene 160 zusammenfallen.

Wenn das Ophthalmoskopie-Vorsatzmodul 114 in die Beobachtungsstrahlengänge 106, 107 des Ophthalmo-Operationsmikroskopsystems 100 eingeschwenkt ist, gewährleistet ein schaltbares System zur Strahlvertauschung und Bildumkehr 108 im Operationsmikroskop 101, dass in dem Einblicktubus 102 des Operationsmikroskops 101 ein aufrechtes Bild des Augenhintergrunds 121 eines Patientenauges 120 beobachtet werden kann.

Die Reduzierlinse 115 im Ophthalmoskopie-Vorsatzmodul 114 verstärkt die Brechkraft des Hauptobjektivsystems 103 vom Operationsmikroskop 101. Sie bewirkt eine Verlagerung der Fokusebene der Beobachtungsstrahlengänge 106, 107 des Operationsmikroskops 101 hin zum Hauptobjektivsystem 103 des Operationsmikroskops 101.

Das Schwenkgelenk 113 ermöglicht, das Ophthalmoskopie-Vorsatzmodul 114 um die Achse 130 entsprechend dem Doppelpfeil 131 in die Beobachtungsstrahlengänge 106, 107 des Operationsmikroskops ein- und auszuschwenken. Die Brechkraft und Anordnung der Reduzierlinse 115 im Ophthalmoskopie-Vorsatzmodul 114 sind so gewählt, dass bei Ausschwenken des Ophthalmoskopie-Vorsatzmoduls 114 aus den Beobachtungsstrahlengängen 106, 107 deren Fokusebene um etwa 2,5 cm in Richtung des untersuchten Patientenauges 120 verlagert wird. Dies ermöglicht, dass bei ausgeschwenktem Ophthalmoskopie-Vorsatzmodul 114 mit dem Ophthalmo-Operationsmikroskopsystem 100 die Linse 140 des Patientenauges 120 scharf gesehen werden kann, ohne dass das Operationsmikroskop 101 nachfokussiert werden muss.

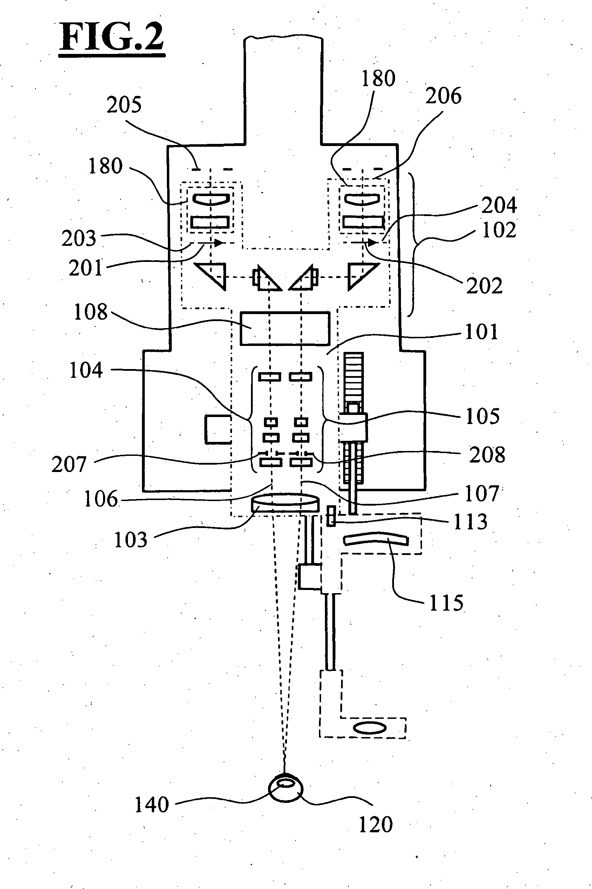

Die Fig. 2 zeigt unter Verwendung gleicher Bezugszeichen wie in Fig. 1 das Ophthalmo-Operationsmikroskopsystem 100 mit einem aus den Beobachtungsstrahlengängen geschwenkten Ophthalmoskopie-Vorsatzmodul 114. Hier ist das System zur Strahlvertauschung und Bildumkehr 108 ausgeschaltet. So kann der vordere Augenabschnitt 140 des Patientenauges 120 aufrecht dargestellt werden.

Das Operationsmikroskop 101 erzeugt mit dem Hauptobjektivsystem 103 und dem pankratischen Vergrößerungssystem 104, 105 im linken Beobachtungsstrahlengang 106 und im rechten Beobachtungsstrahlengang 107 jeweils ein Zwischenbild 201, 202 des Objekts, das mit Hilfe der Okulare 180 betracht werden kann.

Das vom Objekt in das Operationsmikroskop 101 einfallende Licht wird von den Gerätepupillen 207, 208 begrenzt, die im Bereich der pankratischen Vergrößerungssysteme 104, 105 liegen. Die Gerätepupillen 207, 208 werden durch den Tubus 102 und die Okulare 180 in die Austrittspupillen 205, 206 abgebildet. Die Lage der Austrittspupillen 205, 206 beim Operationsmikroskop 101 sind an die typische Kopfhaltung, die eine Person beim Einblick in den Binokulartubus 102 einnimmt, angepasst. Die Position und die Größe der Gerätepupille 207 und die Position und Größe der Austrittspupillen 205, 206 sind durch eine geometrische Abbildung verknüpft, die durch die optischen Elemente im Operationsmikroskop festgelegt ist. Die Austrittspupillen 205, 206 sind ein durch die Linsen im Operationsmikroskop bewirktes Bild der Gerätepupille 207.

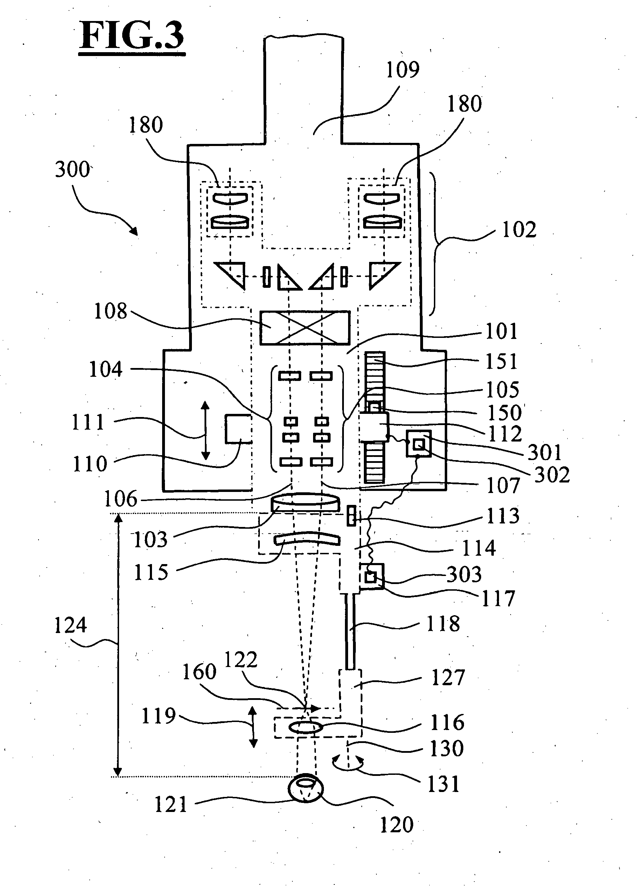

Die Fig. 3 zeigt ein Ophthalmo-Operationsmikroskopsystem 300, dessen Aufbau grundsätzlich dem Ophthalmo-Operationsmikroskopsystem 100 aus Figur 1 entspricht.

Einander entsprechende Baugruppen beim Ophthalmo-Operationsmikroskopsystem 100 aus Fig. 1 und 300 aus Fig. 3 sind mit gleichen Bezugszeichen versehen. Anders als das Ophthalmo-Operationsmikroskopsystem 100 aus Fig. 1 ist beim Ophthalmo-Operationsmikroskopsystem 300 zur Kopplung des Antriebs von Operationsmikroskop 101 und Ophthalmoskopie-Vorsatzmodul 114 keine mechanische, sondern eine elektrische Kopplung 301 vorgesehen. Die Kopplung 301 enthält ein Steuergerät 302, dem Signale des motorischen Antriebs 112 der Fokussiereinrichtung 110 zugeführt werden. Diese Signale werden in dem Steuergerät 302 verarbeitet, um ein Motorsteuersignal an einen Elektromotor 303 abzugeben, der in das Ophthalmoskopie-Vorsatzmodul 114 integriert ist. Dieser Elektromotor 303 wirkt auf den Antrieb 117 der Ophthalmoskopielupenhalterung 127, um diesen entsprechend am Doppelpfeil 119 über einem Patientenauge 120 bewegen zu können.

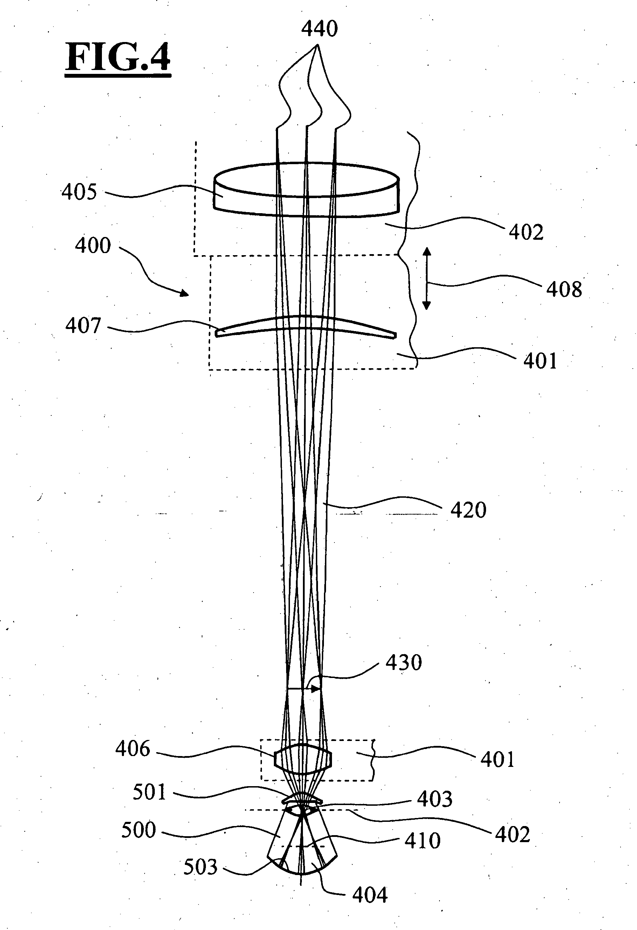

Die Fig. 4 zeigt einen Abschnitt 400 des Ophthalmo-Operationsmikroskopsystems 100 aus Fig. 1 bzw. 300 aus Fig. 3. Das Ophthalmoskopie-Vorsatzmodul 401 ist in die Beobachtungsstrahlengänge des Operationsmikroskops 402 eingeschwenkt. Hierdurch werden die Gerätepupillen 207, 208 aus Fig. 2 in eine neue Gerätepupille 403 abgebildet. Die Gerätepupille 403 ist an die Pupille des untersuchten Patientenauges 500 angepasst, das heißt, die Position der Gerätepupille 403 entspricht der Position der Pupille des Patientenauges 500, wobei die Gerätepupille 403 in der Pupille des Patientenauges 500 liegt.

Zur Einstellung des Ophthalmo-Operationsmikroskopsystems 100 aus Fig. 1 für die Untersuchung des Hintergrunds eines Patientenauges sind daher in einem ersten Schritt bei bekannten Einstellungen von Reduzierlinse 407, Ophthalmoskopierlupe 406 im Bezug auf das Hauptobjektivsystem 405 des Operationsmikroskops 402 durch Heben und Senken des Operationsmikroskops 402 entsprechend dem Doppelpfeil 408 die Gerätepupille 403 mit der Pupille des Patientenauges 500 in Deckung zu bringen. Dies entspricht einem durch die Einstellung des Systems vorgegebenem festen Abstand der Ophthalmoskopierlupe 406 von der Cornea 501 des Patientenauges 500, da der Abstand der Pupillenebene 502 eines Patientenauges 500 vom Scheitelpunkt der Cornea 501 etwa 2 mm beträgt. Die in Fig. 4 gezeigte Einstellung des Ophthalmo-Operationsmikroskopsystems ermöglicht noch keine scharfe Abbildung des Augenhintergrunds des Patientenauges 500. Das System ist auf eine Objektebene 410 fokussiert, die mitten im Glaskörper 504 des Patientenauges 500 liegt. Fig. 4 zeigt einen Abschnitt der abbildenden Lichtstrahlen 420 aus einem der beiden binokularen Beobachtungsstrahlengänge im Ophthalmo-Operationsmikroskopsystem 100 aus Fig. 1. Die Lichtstrahlen durchsetzen die Ophthalmoskopierlupe 406 über deren gesamten Querschnitt und bewirken zwischen Ophthalmoskopierlupe 406 und Reduzierlinse 407 eine Zwischenabbildung 430. Die Lichtstrahlen 420 von der Zwischenabbildung 430 werden durch die Reduzierlinse 407 und das Hauptobjektivsystem in einen parallelen Beobachtungsstrahlengang 440 überführt.

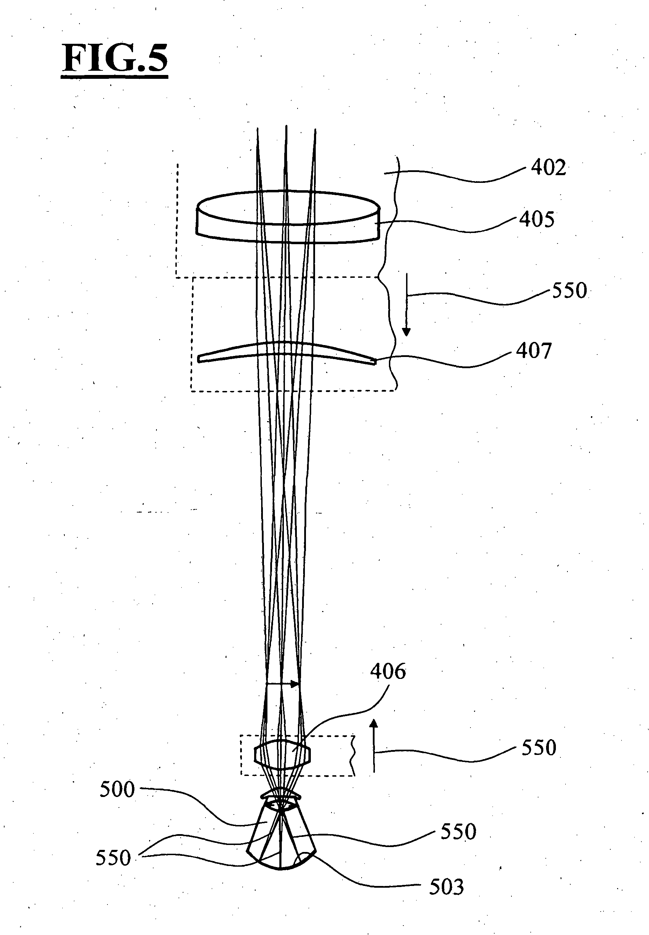

Um das in Fig. 4 gezeigte System auf den Augenhintergrund scharf zu stellen, gilt es, den Abstand der Ophthalmoskopierlupe 406 vom Hauptobjektivsystem 405 und der Reduzierlinse 407 bei gleichbleibender Position der Ophthalmoskopierlupe 406 in Bezug auf die Cornea 501 des Patientenauges 500 zu verringern. Dies ist anhand von Fig. 5 erläutert: Das Operationsmikroskop 402 wird hierzu in Richtung des Pfeils 550 abgesenkt. Gleichzeitig wird dabei die Ophthalmoskopierlupe 406 in Richtung des Pfeils 560 relativ zum Operationsmikroskop 402 bewegt.

Die aufeinander abgestimmte Bewegung von Operationsmikroskop 402 und Antrieb der Ophthalmoskopierlupe 306 wird durch die in Fig. 1 gezeigte Kopplung 123 des Antriebs der Ophthalmoskopierlupe 115 mit dem Antrieb 112 zur Verstellung des Operationsmikroskop 101 bewirkt. Die gegenläufige Bewegung von Operationsmikroskop 402 und Ophthalmoskopierlupe 406 führt zwangsläufig zu einer Geräteeinstellung, wie sie in Fig. 5 gezeigt ist. Diese Einstellung ist optimal für die Untersuchung des Hintergrundes 504 eines Patientenauges 500, da hier das Sehfeld nicht von der Pupille des Patientenauges 500 begrenzt wird.

Bei dem erfindungsgemäßen Ophthalmo-Operationsmikroskopsystem ist eine Positionssteuerung des Antriebes 112 für das Operationsmikroskop 101 aus Fig. 1 und des Antriebes 117 für die Ophthalmoskopierlupe 116 vorgesehen. Die Kopplung der Antriebe 112, 117 ist dabei entsprechend einer nichtlinearen Abhängigkeit ausgeführt, die gewährleistet, dass sich bei Verstellen von Operationsmikroskop 101 und Ophthalmoskopie-Vorsatzmodul 114 die Lage der Gerätepupille nicht verändert. So wird während einer ophthalmologischen Operation an einem Patientenauge eine stets ideale Anpassung von Gerätepupille des Ophthalmo-Operationsmikroskopsystems an die Pupille des Patientenauges gewährleistet. Bei einer solchen Kopplung besteht eine nichtlineare Beziehung der Verstellgeschwindigkeit für das Operationsmikroskop und die Ophthalmoskopierlupe.

Indem in das Operationsmikroskopsystem ein Sensor zur Messung des Abstands vom Operationsmikroskop 101 aus Fig. 1 zum Patientenauge 120 integriert wird, kann mittels einer Verstellanzeige der Operateur die stets günstige Systemeinstellung, auf die das Ophthalmo-Operationsmikroskopsystem eingestellt werden sollte, angezeigt werden. So können unerwünschte Bewegungen des Patientenauges während einer Operation ausgeglichen werden.

Es ist auch möglich, einen solchen Sensor mit einer Regeleinrichtung zu kombinieren, die gewährleistet, dass das Ophthalmo-Operationsmikroskopsystem bei Bewegungen des Patientenauges automatisch die günstigste Systemeinstellung vornimmt.

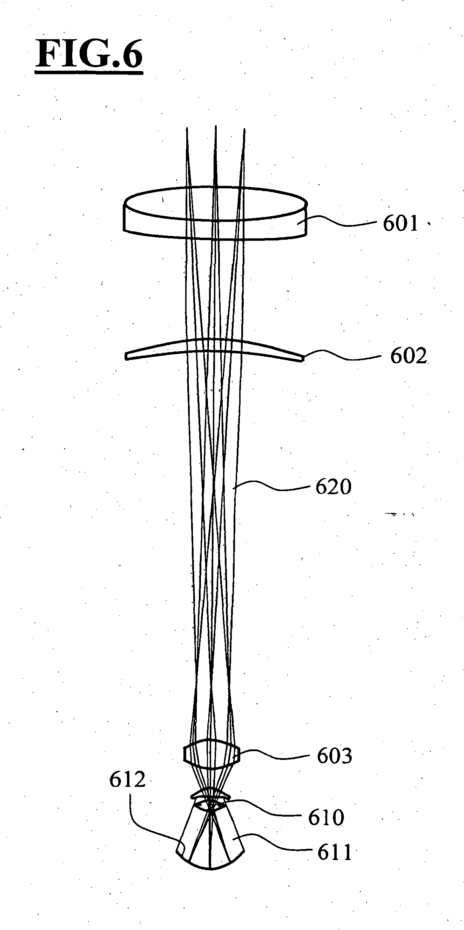

Die Fig. 6 zeigt demgegenüber einen Abschnitt eines Ophthalmo-Operationsmikroskopsystems 600 mit Hauptobjektivsystem 601, Reduzierlinse 602 und Ophthalmoskopierlupe 603. Das Ophthalmo-Operationsmikroskopsystem 600 ermöglicht mit einem Beobachtungsstrahlengang 620 eine scharfe Abbildung des Hintergrunds 612 des Patientenauges. Da jedoch die Gerätepupille 604 des Systems nicht an die Pupille 610 des Patientenauges 611 angepasst ist, wird von der Ophthalmoskopierlupe 603 im Vergleich zu der anhand von Fig. 5 erläuterten Einstellung des erfindungsgemäßen Ophthalmo-Operationsmikroskopsystems nur ein vergleichsweise kleiner Teil des Augenhintergrunds 612 abgebildet.

## Patentansprüche

1. Ophthalmo-Operationsmikroskopsystem (100, 300) mit
- einem Operationsmikroskop (101, 402);
- einer Trägereinrichtung (109), in der Operationsmikroskop (101, 402) höhenverstellbar aufgenommen ist, um einen Arbeitsabstand (124) zwischen dem Operationsmikroskop (101, 402) und einem Patientenauge (120, 400) einstellen zu können;
- einem ersten Antrieb (112) zur Höhenverstellung des Operationsmikroskops (101, 402);
- einem Ophthalmoskopie-Vorsatzmodul (114, 401), das eine verstellbare Ophthalmoskopierlupe (116, 406) umfasst, um einen Abstand zwischen Ophthalmoskopierlupe (116, 406) und Operationsmikroskop (101, 402) einstellen zu können;
- einem zweiten Antrieb (117) zur Verstellung der Ophthalmoskopierlupe (116, 406)
- eine Antriebskopplung (123, 301), die den ersten Antrieb (112) zur Höhenverstellung des Operationsmikroskops (101, 402) mit dem zweiten Antrieb (117) zur Verstellung der Ophthalmoskopierlupe (116, 406) koppelt,
**dadurch gekennzeichnet, dass** die Antriebskopplung (123, 301) den ersten Antrieb (112) zur Höhenverstellung des Operationsmikroskop (101, 402) mit dem zweiten Antrieb (117) zur Verstellung der Ophthalmoskopierlupe (116, 306) derart koppelt, dass Operationsmikroskop (101, 402) und Ophthalmoskopierlupe (116, 306) gleichzeitig in entgegengesetzter Richtung bewegbar sind, wobei die Antriebskopplung (123, 301) den ersten Antrieb (112) zur Höhenverstellung des Operationsmikroskops (101, 402) mit dem zweiten Antrieb (117) zur Verstellung der Ophthalmoskopierlupe (116, 406) in einer nichtlinearen Abhängigkeit koppelt, dass die Gerätepupille (403) des Systems aus Operationsmikroskop (101, 302) und Ophthalmoskopierlupe (116, 406) beim Verstellen des Operationsmikroskops (101, 402) und Ophthalmoskopie-Vorsatzmoduls (114, 401) raumfest ist.

2. Ophthalmo-Operationsmikroskopsystem nach Anspruch 1, **dadurch gekennzeichnet, dass** der erste Antrieb (112) als motorischer Antrieb ausgebildet ist.

3. Ophthalmo-Operationsmikroskopsystem nach einem der Ansprüche 1 bis 2, **dadurch gekennzeichnet, dass** der zweite Antrieb (117) als motorischer Antrieb ausgebildet ist.

4. Ophthalmo-Operationsmikroskopsystem nach Anspruch 3, **dadurch gekennzeichnet, dass** die Kopplung als elektronische Kopplung (301) ausgebildet ist.

5. Ophthalmo-Operationsmikroskopsystem nach einem der Ansprüche 1 bis 4, **dadurch gekennzeichnet, dass** das Ophthalmoskopie-Vorsatzmodul (114, 401) eine Reduzierlinse (115, 407) umfasst.

6. Ophthalmo-Operationsmikroskopsystem nach einem der Ansprüche 1 bis 5, **dadurch gekennzeichnet, dass** ein System zur Strahlvertauschung und Bildumkehr (108) vorgesehen ist.

7. Ophthalmo-Operationsmikroskopsystem nach einem der Ansprüche 1 bis 6, **dadurch gekennzeichnet, dass** das Ophthalmoskopie-Vorsatzmodul (114, 401) in die Beobachtungsstrahlengänge (106, 107) des Operationsmikroskops (101, 402) ein- und ausgeschwenkt werden kann.

## Claims

1. Ophthalmic operation microscope system (100, 300) comprising:
- an operation microscope (101, 402);
- a support apparatus (109), in which operation microscope (101, 402) is held in a height-adjustable manner so as to be able to set a working distance (124) between the operation microscope (101, 402) and a patient's eye (120, 400);
- a first drive (112) for adjusting the height of the operation microscope (101, 402);
- an ophthalmoscopy attachment module (114, 401) which comprises an adjustable ophthalmoscopy magnifier (116, 406) so as to be able to set a distance between ophthalmoscopy magnifier (116, 406) and operation microscope (101, 402);
- a second drive (117) for adjusting the ophthalmoscopy magnifier (116, 406);
- a drive coupling (123, 301) which couples the first drive (112) for adjusting the height of the operation microscope (101, 402) with the second drive (117) for adjusting the ophthalmoscopy magnifier (116, 406),
**characterized in that** the drive coupling (123, 301) couples the first drive (112) for adjusting the height of the operation microscope (101, 402) with the second drive (117) for adjusting the ophthalmoscopy magnifier (116, 306) in such a way that operation microscope (101, 402) and ophthalmoscopy magnifier (116, 306) are movable simultaneously in opposite directions, wherein the drive coupling (123, 301) couples the first drive (112) for adjusting the height of the operation microscope (101, 402) with the second drive (117) for adjusting the ophthalmoscopy magnifier (116, 406) with a non-linear dependence so that the instrument pupil (403) of the system of operation microscope (101, 302) and ophthalmoscopy magnifier (116, 406) is fixed in space when adjusting the operation microscope (101, 402) and ophthalmoscopy attachment module (114, 401).

2. Ophthalmic operation microscope system according to Claim 1, **characterized in that** the first drive (112) is embodied as a motor-driven drive.

3. Ophthalmic operation microscope system according to either of Claims 1 and 2, **characterized in that** the second drive (117) is embodied as a motor-driven drive.

4. Ophthalmic operation microscope system according to Claim 3, **characterized in that** the coupling is embodied as an electronic coupling (301).

5. Ophthalmic operation microscope system according to one of Claims 1 to 4, **characterized in that** the ophthalmoscopy attachment module (114, 401) comprises a reduction lens (115, 407).

6. Ophthalmic operation microscope system according to one of Claims 1 to 5, **characterized in that** provision is made for a system for ray displacement and image reversal (108).

7. Ophthalmic operation microscope system according to one of Claims 1 to 6, **characterized in that** the ophthalmoscopy attachment module (114, 401) can be pivoted into, and out of, the observation beam paths (106, 107) of the operation microscope (101, 402).

## Revendications

1. Système (100, 300) de microscope pour opération ophtalmologique, présentant
un microscope opératoire (101, 402) ;
un dispositif de support (109) dans lequel le microscope opératoire (101, 402) est repris à hauteur réglable pour pouvoir régler la distance de travail (124) entre le microscope opératoire (101, 402) et l'oeil (120, 400) d'un patient ;
un premier entraînement (112) qui assure le réglage en hauteur du microscope opératoire (101, 402) ;
un module (114, 401) de lunette d'ophtalmoscopie qui comporte une loupe ajustable d'ophtalmoscopie (116, 406) permettant d'établir la distance entre la loupe (116, 406) d'ophtalmoscopie et le microscope opératoire (101, 402) ;
un deuxième entraînement (117) de réglage de la loupe d'ophtalmoscopie (116, 406) ;
un accouplement d'entraînement (123, 301) qui accouple le premier entraînement (112) de réglage en hauteur du microscope opératoire (101, 402) au deuxième entraînement (117) qui règle la loupe d'ophtalmoscopie (116, 406),
**caractérisé en ce que** l'accouplement d'entraînement (123, 301) accouple le premier entraînement (112) de réglage en hauteur du microscope opératoire (101, 402) au deuxième entraînement (117) qui règle la loupe d'ophtalmoscopie (116, 306) de telle sorte que le microscope opératoire (101, 402) et la loupe d'ophtalmoscopie (116, 306) puissent être déplacés simultanément dans des directions opposées, l'accouplement d'entraînement (123, 301) accouplant le premier entraînement (112) de réglage en hauteur du microscope opératoire (101, 402) au deuxième entraînement (117) qui règle la loupe d'ophtalmoscopie (116, 406) selon une dépendance non linéaire, de telle sorte que la pupille (403) du système constitué du microscope opératoire (101, 302) et de la loupe d'ophtalmoscopie (116, 406) soit fixe dans l'espace lors du réglage du microscope opératoire (101, 402) et du module de lunette d'ophtalmoscopie (114, 401).

2. Système de microscope pour opération ophtalmologique selon la revendication 1, **caractérisé en ce que** le premier entraînement (112) est configuré comme entraînement motorisé.

3. Système de microscope pour opération ophtalmologique selon l'une des revendications 1 et 2, **caractérisé en ce que** le deuxième entraînement (117) est configuré comme entraînement motorisé.

4. Système de microscope pour opération ophtalmologique selon la revendication 3, **caractérisé en ce que** l'accouplement est configuré comme accouplement électronique (301).

5. Système de microscope pour opération ophtalmologique selon l'une des revendications 1 à 4, **caractérisé en ce que** le module (114, 401) de lunette d'ophtalmoscopie comporte une lentille réductrice (115, 407).

6. Système de microscope pour opération ophtalmologique selon l'une des revendications 1 à 5, **caractérisé en ce qu'**il présente un système de remplacement de rayon et d'inversion d'image (108).

7. Système de microscope pour opération ophtalmologique selon l'une des revendications 1 à 6, **caractérisé en ce que** le module (114, 401) de lunette d'ophtalmoscopie peut être placé dans le parcours des rayons d'observation (106, 107) du microscope opératoire (101, 402) et en être retiré.
